# EUROPEAN PATENT APPLICATION

(11) **EP 4 302 615 A1**
(43) Date of publication of application: **10.01.2024**
(21) Application number: 22020322.8
(22) Date of filing: 07.07.2022
(51) Int. Cl.: A23L 33/105, A23K 20/10, A23K 50/30, A23K 50/75

(54) **FOOD SUPPLEMENT COMPRISING NEOHESPERIDIN DIHYROCHALCONE AND PEPPERMINT OIL**

(71) Applicant: ADM International Sarl, 1180 Rolle (CH)
(72) Inventor: Blanchard, Alexandra, 01630 Challex (FR); Khelil-Arfa, Hajer, 74160 Saint Julien en Genevois (FR); Ionescu, Catherine, 01420 Corbonod (FR)
(74) Representative: Stona, Daniel

(57) **Abstract**

The invention relates to a food product for animals which can be used as a prophylactic measure to reduce the number of *Campylobacter jejuni* in hosts like poultry or swine.

More specifically the invention is a food supplement comprising neohesperidin dihydrochalcone (NHDC) and peppermint oil (PMO).

The invention also pertains to a feed comprising such a food supplement as well as to its use to reduce campylobacter carriage by poultry or swine and to a method of reducing the risk of *Campylobacter spp.* infection in humans.

## Description

The invention relates to a food supplement comprising neohesperidin dihyrochalcone and peppermint oil.

The invention also pertains to a feed comprising such a food supplement as well as to its use to reduce campylobacter carriage by poultry or swine and to a method of reducing the risk of *Campylobacter spp.* infection in humans.

### Background of the invention

Campylobacteriosis is a zoonosis of concern as this is a huge human public health issue in Europe, USA, Australia and New Zealand. The disease, of food origin, is mainly due to chicken meat contamination by *Campylobacter spp.* which can be transferred to humans through consumption and handling of meat. The main campylobacter species are *C. jejuni* and *C. coli* which can induce clinical signs such as diarrhea, abdominal pain, headache, nausea, vomiting and in worst cases, arthritis, irritable bowel syndrome (IBS) and Guillain-Barre syndrome (GBS). As a consequence, the asymptomatic carriage of *Campylobacter spp.* by poultry is a major worry. C. *jejuni* colonize the broiler chicken digestive tract but is generally not pathogenic for its host in normal conditions.

The use of antibiotics is the main way to reduce asymptomatic carriage of the bacteria and the industry is looking for alternatives.

On the other hand some plant based compounds have shown some positive impacts on *Campylobacter jejuni* either *in vitro* through the reduction of cell invasion or through a reduction of asymptomatic carriage. Van Alphen et al., 2012 [1] observed that carvacrol had an effect on the motility of *Campylobacter jejuni* at doses of 0.25 mM and reduced cell invasion at a dose of 0.2 mM.

Others have looked at the positive effects of curcumin as presented by Lobo de Sa et al., 2019 [2] who highlighted the capacity of curcumin to mitigates the immune-induced epithelial disfunction by *Campylobacter jejuni* in HT-29 cells co-cultured with immune cells at doses of 50 µmoles.

The effect of a peppermint oil has been investigated on the stress response and virulence of the application of the product on *Campylobacter jejuni* NCTC 11168 cell at sublethal doses of 50 and 150 µg/ml selected between the MIC of 100 µg/ml and the MBC of 400 µg/ml measured by the Kovacs et al., (2019) [3].

However even if these products have shown some positive effects in *in vitro* tests, their effect *in vivo* on the asymptomatic carriage has not been performed.

### Summary of the Invention

The main goal of the invention is to propose a food product for animals which can be used as a prophylactic measure to reduce the number of *Campylobacter jejuni* in hosts like poultry or swine.

The invention more specifically relates to a food supplement according to item 1 below:
1. A food supplement comprising neohesperidin dihydrochalcone (NHDC) and peppermint oil (PMO), preferably mixed.

Additional advantageous features of the food supplement as defined in item 1 above are specified in items 2 to 6 below:
2. The food supplement of item 1, wherein the weight ratio of NHDC/PMO ranges from 0.5 to 1.
3. The food supplement of item 2, wherein the weight ratio of NHDC/PMO ranges from 0.65 to 0.85.
4. The food supplement of item 3, wherein the weight ratio of NHDC/PMO is 0.75.
5. The food supplement of any one of items 1-4, wherein NHDC and PMO are under solid form.
6. The food supplement of any one of items 1-4, wherein NHDC and PMO are under liquid form.

The invention also deals with a feed according to item 7 below:
7. A feed comprising the food supplement of the above items 1 to 6.

Additional advantageous features of the feed as defined in item 7 above are specified in items 8 to 10 below:
8. The feed of item 7, comprising from 10 to 25 ppm of the food supplement of items 1 to 6.
9. The feed of item 8, comprising from 15 to 20 ppm of the food supplement of items 1 to 6.
10. The feed of item 9, comprising 17.5 ppm of the food supplement of items 1 to 6.

According to another aspect, the invention pertains to the use as defined in item 11 below:
11. Use of the food supplement of items 1 to 6 or feed of items 7 to 10 to reduce the load of *Campylobacter spp.* in poultry.

According to another aspect, the invention pertains to the use as defined in item 12 below:
12. Use of the food supplement of items 1 to 6 or feed of items 7 to 10 to reduce the load of *Campylobacter spp.* in swine.

According to another aspect, the invention also concerns a method as defined in item 13 below:
13. A method of reducing the risk of *Campylobacter spp.* infection in humans comprising feeding the humans with poultry that has been fed with the food supplement according to items 1 to 6 or feed according to items 7 to 10.

According to another aspect, the invention also concerns a method as defined in item 14 below:
14. A method of reducing the risk of *Campylobacter spp.* infection in humans comprising feeding the humans with swine that has been fed with the food supplement according to items 1 to 6 or feed according to items 7 to 10.

According to a further aspect, the invention relates to a composition comprising neohesperidin dihydrochalcone (NHDC) and peppermint oil (PMO) for use in reducing the load of *Campylobacter spp.* in poultry and/or swine.

According to a further aspect, the invention pertains to a method of reducing the load of *Campylobacter spp.* in poultry and/or swine by feeding the same with a composition comprising neohesperidin dihydrochalcone (NHDC) and peppermint oil (PMO).

The combination of peppermint oil and NHDC in a broiler chicken or piglet diet can therefore reduce their *Campylobacter* carriage and thus potentially reduce the occurrence of campylobacteriosis in human. Consequently, the use of antibiotics in poultry or swine can be limited which in turn reduces the development of antibiotic resistance.

Other features and advantages of the invention will now be described in detail in the following description, which refers to the appended figures that show:
[Fig. 1]: a schematic representation of the experimental *in vitro* design;
[Fig. 2]: the *in vitro* attachment results (combined results from both cell lines);
[Fig. 3]: the *in vivo* trial design timeline representation; and
[Fig. 4]: the Campylobacter bacteriological counts at D14 post infection in caeca expressed in log CFU/ml, left panel or in log CFU/organ right panel.

### Detailed description of the invention

The inventors have first carried out a selection of different compounds to be included in feeds as preventive agents.

In a first step, an *in vitro* screening looking at the attachment capacity of *Campylobacter jejuni* on 2 cells lines after pre-treatment with the different compounds was performed.

In a second step, the 2 best products elected from the *in vitro* tests were tested in a challenge test on broiler chickens looking at asymptomatic *Campylobacter jejuni* carriage levels in the ceca which is known to be representative of carcass contamination.

With the above tests, first *in vitro* and then *in vivo,* the inventors have observed at the end of the broiler chicken or piglet production cycle a significant reduction of 1 log of *Campylobacter jejuni* in broiler chickens using a combination of neohesperidin dihydrochalcone (NHDC) and peppermint oil (PMO).

According to a preferred embodiment, the weight ratio of NHDC/PMO in the food supplement ranges from 0.5 to 1, more preferably from 0.65 to 0.85. Ideally, the weight ratio of NHDC/PMO is about 0.75. NHCH and PMO are preferably homogeneously mixed together.

As NHDC is a solid raw material and PMO is a typical liquid raw material, there is a need for additional product formulation for a good in feed inclusion. Therefore, either the PMO is formulated in order to produce solid particles or the NHDC is solubilized in order to be able to combine the 2 raw materials together with a closer physical state in order to get a homogeneous food supplement.

Different formula can be produced. For the transformation of PMO into a particulate in a first option the PMO can be adsorbed/absorbed onto a carrier which can be selected from any porous carrier which can be selected from silicon dioxide, sepiolite, diatomaceous earth, vermiculite, zeolite, porous starch. In a second option PMO can be processed to produce particles through introduction in a liquid matrix which can be either solidified through the use of atomization or solidification. The liquid matrixes can include modified starch, cellulose derivatives, maltodextrin, gums, natural gum, waxes in combination with amphiphilic molecules, fats or hydrogenated fats to be spray dried, spray granulated or sprayed chilled.

For the transformation of NHDC into a liquid product, the product can be solubilized, solvated or emulsified in water or solvents to be compatible with PMO.

In case, PMO is to be transformed to a solid state, the obtained product can be mixed with NHDC directly or with an additional diluent. Another possibility is to agglomerate NHDC to the PMO particles during the process using possibly spray agglomeration.

The food complement may be added to the feed of the animal, preferably poultry or swine. In that case, the feed preferably comprises from 10 to 25 ppm of food supplement, more preferably from 15 to 20 ppm of food supplement. Ideally the feed comprises about 17.5 ppm of food supplement.

### Experiments

Two sets of trials have been performed:
- an *in vitro* data looking at attachment / penetration of *Campylobacter jejuni* in 2 cell lines and
- an *in vivo* challenge test in broiler chickens.

### a) In vitro screen test

*Campylobacter jejuni* MDR1 new (strain presenting multi-resistance) was used for the *in vitro* challenge. The bacteria was cultured in micro-aerophile jar.

The following medias were used for the tests:
- William's E media (BioWhittaker ref. BE12-761F)
- Dulbecco's Modified Eagle Medium (DMEM) 4.5 g glucose /L (Gibco ref. 41965-039)
- Fetal Bovine Serum (FBS) (Sigma ref. F7524)
- L-glutamine 200 mM (Gibco ref. 25030-024)
- Gentamicine 50 mg/mL (Gibco ref. 1570037)
- PBS (Sigma ref. D8662)
- Ethanol 96° (Carlo-Erba ref. 528151)
- Blood agar

2 cell lines were used for the *in vitro* tests: HT-29 which is a human colorectal adenocarcinoma cell line with epithelial morphology and LHM which is a *Gallus gallus* hepatho-carcinoma cell line with epithelial morphology.

The 2 cell lines were cultured in the following respective media. LHM was cultured in 10% fetal bovine serum (FBS) or FBS and William's E media, while HT-29 was cultured in a media including 1% glutamine, 10% FBS and DMEM 4.5 g glucose/L.

**Table 1: Listing of tested products, their combinations and associated doses**

| **Test** | **Products names and combinations** | **Dose** |
|---|---|---|
| **1.** | NHDC. Ref 4007817G002 purity minimum 96% | 15 mg/L |
| **2** | Peppermint oil^{∗}, ref 2018CROP | 20 mg/L |
| **3.** | Peppermint oil^{∗} + NHDC | 20 mg/L + 15 mg/L |

| | | |
|---|---|---|
| ^{∗}The used peppermint oil was an peppermint essential oil or a peppermint extract which main components are included in table 2 | | |

**Table 2: Specification of peppermint oils or extracts**

| **Molecules** | **Minimum weight %** | **Maximum weight %** |
|---|---|---|
| Eucalyptol | 2.5 | 7 |
| Menthone | 10 | 30 |
| Menthol | 20 | 60 |
| Pulegone | 0.0 | 4 |
| L-menthyl acetate | 0.5 | 8 |
| Germacene D | 0.2 | 2 |
| Menthofuran | 3 | 7 |

The effects of the compounds were compared to a control based on ethanol 96% at 10 µl. All tests were performed in microwells.

The *in vitro* challenge for the 2 cell lines was the following:
- The *Campylobacter jejuni* charge was set a 8 log of CFU which is the dose used for the *in vivo* experiments.
- The multiplicity of infection (MOI) was defined as the ratio between the number of bacteria and the number of potential host cells or number of bacteria used for the inflection (CFU)/number of host cell (UFC) was set at 100.
- Contact time was set at 30 min.
- Cells in confluence were infected by the bacterial suspension.

The tests were performed to reach 6 replicates per treatment and cell line and included at minima 1 duplicate per day in case of different testing dates.

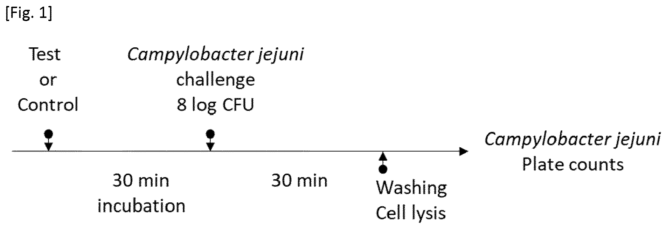

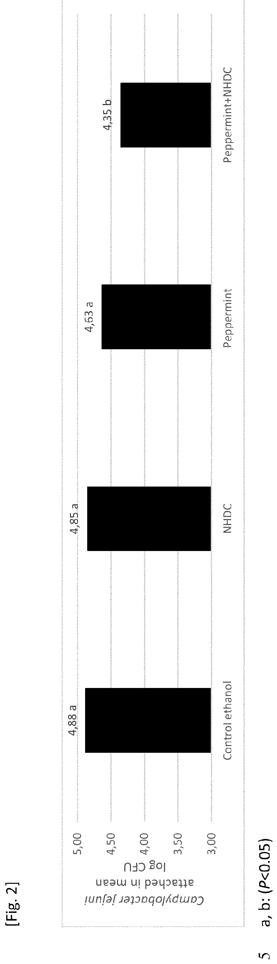

The scheme of the *in vitro* experimental design main time settings is shown in Figure 1. The *Campylobacter jejuni* plate counts were then analyzed and the average of the log CFU/well for the 2 cell lines combined shown in Figure 2.

From the *in vitro* tests, it can be observed that each product individually did not induce any significant reduction of the attachment penetration of *Campylobacter jejuni* in the cells but that the combination of peppermint with NHDC induced a significant reduction of the *Campylobacter jejuni* attachment or penetration in the cell highlighting the synergistic effect of the use of the combinations of products.

### b) In vivo tests

The combination obtained above was further tested *in vivo.* The description of the *in vivo* challenge trial is presented hereinafter.

The tested products and their concentrations in the feed are disclosed in Table 3.

**Table 3: Target inclusion levels of the actives or plant extract in the feed**

| **Product name** | **Raw material 1** | **Concentration in the feed (ppm)** | **Raw material 2** | **Concentration in the feed (ppm)** |
|---|---|---|---|---|
| **PIP-A** | NHDC | 7.5 | Peppermint oil^{∗∗} | 10 |

| | | | | |
|---|---|---|---|---|
| ^{∗∗} same as above | | | | |

The experiment lasted 35 days during which the growth of the chickens was followed. The dietary treatments have been administered to the broiler chickens through feed from day 0 to day 35.

The bacterial inoculation have been performed at day 21. Autopsies and cecal analyses have been performed at day 4, 10 and 14 post-inoculation corresponding to D25, 31 and 35 of age, to follow cecal colonization in function of treatments (7/treatment).

The following experimental design have been applied:

### Pre-trial set-up

(i) egg pick-up at the hatter,
(ii) egg decontamination and incubation during 21 days,

### Trial set-up

(i) arrival of 1 day old chicks in pens on straw,
(ii) J-7 (before challenge) sanitary status check *(Campylobacter* spp., *Salmonella* spp., *Clostridium perfringens,* enteropathogenic *E. coli)* of the chicks on 3 broilers/treatment,
(iii) breeding of the broilers until 21 days (zootechnical follow up, collection and weighing of feeds refusals and weight gain data collection),
(iv) inoculation of C. *jejuni* CJ-MDR1 at 2.07×10⁸ c.f.u in 0.2 ml by oral gavage at 21 days and follow up of carriage until the end of the protocol (zootechnical follow up, weight gain, feed intake of un-supplemented and supplemented feeds,
(v) autopsies at day 4, 10 and 14 post infection (p.i.) of 7 broiler chickens per treatment. At each autopsy, the 2 caeca + tonsils have been sampled for bacterial numeration (analysis performed by the company Inovalys).

### Feeds

The animals have been treated with the product PIP-A: NHDC and peppermint oil incorporated in the feed all over the protocol. The diets were pelleted at a size of 6 mm long x 2.5 mm diameter. The diet raw material composition is indicated in Table 4.

**Table 4: Control Diet composition**

| **Component** | **Basal diet (weight %)** |
|---|---|
| **Corn** | 28.0 |
| **Wheat** | 30.0 |
| **Soybean meal** | 34.2 |
| **Soya Oil** | 4.0 |
| **Calcium carbonate** | 0.9 |
| **Phosphate Dicalcium** | 1.9 |
| **Methionine** | 0.17 |
| **Premix VHT791NE^{∗}** | 0.4 |
| **Salt** | 0.4 |
| **Total** | **100** |

| | |
|---|---|
| ^{∗}anticoccidial SACOX | |

The chickens were fed a single phase diet for the total duration of the trial. The product PIP-A was added on top of the above mentioned basal diet in a diluted form included at a 0.166% extend of the diet, which is not supposed to influence the global dietary composition in order to provide 7.5 ppm of NHDC and 10 ppm of peppermint oil in the supplemented feed.

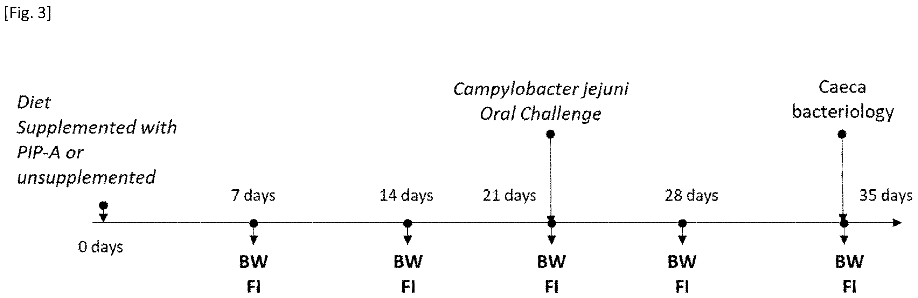

The overall *in vivo* trial design is schematically represented in Figure 3.

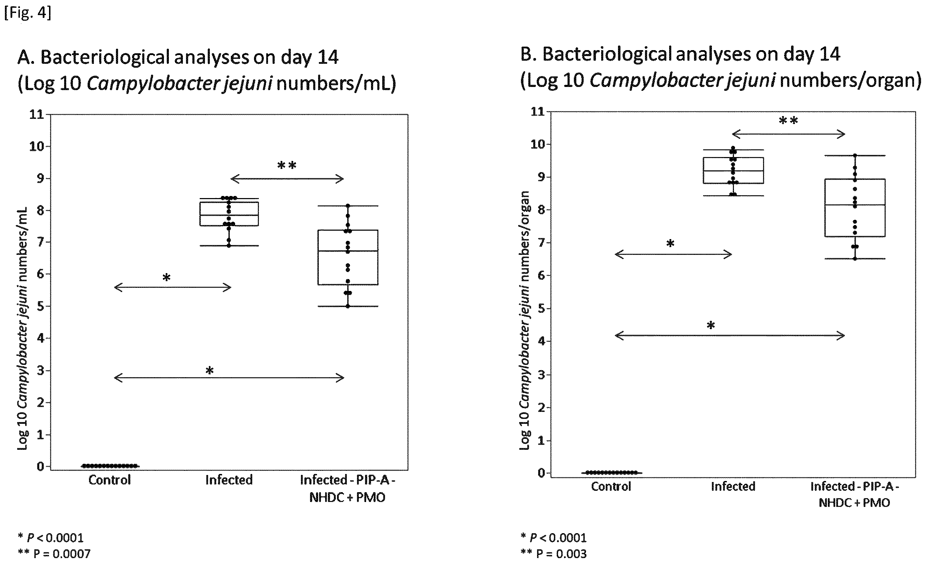

The outcome of the *in vivo* trial and particularly the *Campylobacter spp.* bacteriological counts at the end of the production period (D35 corresponding to D14 post infection) are presented in Figure 4.

The results show that the combination of peppermint with NHDC has a positive impact in significantly reducing the *Campylobacter jejuni* carriage of the chicken in their caeca after 14 days of their challenge by around 1 log the effect being highly significant (P<0.007).

### Examples of formulations of the NHDC/PMO food supplement

### Example 1: powder Formula 1

| Raw material | Weight % |
|---|---|
| NHDC | 0.8 |
| PMO | 1.3 |
| Hydrogenated fat | 11.7 |
| Diluent | 86.2 |
| Total | 100 |

### Example 2: powder Formula 2

| Raw material | Weight % |
|---|---|
| NHDC | 0.5 |
| PMO | 0.6 |
| Hydrogenated fat | 5.4 |
| Silicon dioxide | 1.4 |
| Calcium carbonate | 10.0 |
| Wheat middlings | 82.1 |
| Total | 100 |

### Example 3: powder Formula 3

| Raw material | Weight % |
|---|---|
| NHDC | 0.5 |
| PMO | 0.6 |
| Silicon dioxide | 98.9 |
| Total | 100 |

### Example 4: powder Formula 4

| Raw material | Weight % |
|---|---|
| NHDC | 0.8 |
| PMO | 1.3 |
| Maltodextrin | 87.9 |
| Gum | 10.0 |
| Total | 100 |

### Example 5: liquid Formula 1

| | |
|---|---|
| Raw material | Weight % |
| NHDC | 0.5 |
| PMO | 0.6 |
| GLYCERYL POLYETHYLEN GLYCOL RICINOLEATE | 5.4 |
| Preservative | 0.5 |
| Water | 93.0 |
| Total | 100 |

## Claims

1. A food supplement comprising neohesperidin dihydrochalcone (NHDC) and peppermint oil (PMO).

2. The food supplement of claim 1, wherein the weight ratio of NHDC/PMO ranges from 0.5 to 1.

3. The food supplement of claim 2, wherein the weight ratio of NHDC/PMO ranges from 0.65 to 0.85.

4. The food supplement of claim 3, wherein the weight ratio of NHDC/PMO is 0.75.

5. The food supplement of any one of claims 1-4, wherein NHDC and PMO are under solid form.

6. The food supplement of any one of claims 1-4, wherein NHDC and PMO are under liquid form.

7. A feed comprising the food supplement of claims 1 to 6.

8. The feed of claim 7, comprising from 10 to 25 ppm of the food supplement of claims 1 to 6.

9. The feed of claim 8, comprising from 15 to 20 ppm of the food supplement of claims 1 to 6.

10. The feed of claim 9, comprising 17.5 ppm of the food supplement of claims 1 to 6.

11. Use of the food supplement of claims 1 to 6 or feed of claims 7 to 10 to reduce the load of *Campylobacter spp.* in poultry.

12. Use of the food supplement of claims 1 to 6 or feed of claims 7 to 10 to reduce the load of *Campylobacter spp.* in swine.

13. A method of reducing the risk of *Campylobacter spp.* infection in humans comprising feeding the humans with poultry that has been fed with the food supplement according to claims 1 to 6 or feed according to claims 7 to 10.

14. A method of reducing the risk of *Campylobacter spp.* infection in humans comprising feeding the humans with swine that has been fed with the food supplement according to claims 1 to 6 or feed according to claims 7 to 10.
